Europäisches Patentamt

European Patent Office

Office européen des brevets

(11) Publication number: **0 205 387 B1**

# EUROPEAN PATENT SPECIFICATION

(45) Date of publication of patent specification: **26.02.92**

(51) Int. Cl.⁵: **C12N 5/00**, C12N 5/08, C12M 3/02, A61K 35/14, A61K 45/06

(21) Application number: **86401250.5**

(22) Date of filing: **10.06.86**

(54) Human monocytes cultured in suspension in serum-free medium.

(30) Priority: **11.06.85 US 743570**

(43) Date of publication of application:
**17.12.86 Bulletin 86/51**

(45) Publication of the grant of the patent:
**26.02.92 Bulletin 92/09**

(84) Designated Contracting States:
**AT BE CH DE FR GB IT LI LU NL SE**

(56) References cited:

**BIOLOGICAL ABSTRACTS, vol. 78, 1984, abstract no. 76990, Philadelphia, PA, US; H.C. STEVENSON et al.: "Characterization of biological response modifier release by human monocytes cultured in suspension in serum-free medium", & J IMMUNOL METHODS 70(2): 245-256. 1984**

(73) Proprietor: **THE UNITED STATES OF AMERICA as represented by the Secretary, United States Department of Commerce National Technical Information Service, Office of Government Inventions and Patents, 5285 Port Royal Road Springfield, Virginia 22161(US)**

(72) Inventor: **Colburn Stevenson, Henry, Dr. 10105 Ashwood Dr. Kensington Maryland 20895(US)**

(74) Representative: **Mongrédien, André et al c/o SOCIETE DE PROTECTION DES INVENTIONS 25, rue de Ponthieu F-75008 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BIOLOGICAL ABSTRACTS, vol. 77, 1984, abstract no. 69683, Philadelphia PA, US; P.H.M. DE MULDER et al.: "Characterization of monocyte maturation in adherent and suspension cultures and its application to study monocyte differentiation in hodgkins disease" & CLIN EXP IMMUNOL 54(3): 681-688. 1983

JOURNAL OF LEUKOCYTE BIOLOGY, vol. 36, no. 5, 1984, page 662, H.C. STEVENSON et al.: "Suspension culture of human blood monocytes in serum free medium"

Stevenson et al., Journal of immunological methods, 70(1984), 245-255. Characterisation of biological response modifier release by human monocytes cultured in suspension in serum-free medium

Stevenson et al., Journal of Immunological methods, 70 (1984), 245-255 Characterisation of biological responsemodifier release by human monocytes cultured in suspension in serum-free medium.

## Description

Technical Field

The present invention is related to in vitro culture of purified human monocytes. More particularly, the present invention is related to the culture of purified human monocytes in suspension in a serum-free medium.

Background Art

Mononuclear phagocytes (monocytes) in their various forms have been shown to participate in many critical phases of the mammalian immune response. Monocytes and macrophages are known to be essential for the initiation of immune responses by virtue of their ability to process antigen (Rosenthal, New Engl. J. Med. 303, 1153. 1980), and for their ability to secrete soluble factors such as interleukin 1 (IL-1), colony stimulating factor (CSF), interferon (IFN) and prostaglandin E (PGE) which allow them to function as immunoregulators for a number of immune responses (Epstein, Biology of Lymphokines; Academic Press, NY, pp. 123-152. 1979; Stevenson, The Reticuloendothelial System. A Comprehensive Treatise, Vol. VI: Plenum Press, NY, pp. 79-91. 1982). In addition, monocytes are known to play a critical role as final effector cells in humoral immunity by virtue of the fact that these cells secrete complement components (Nathan, et al, New England J. Med. 303, 623. 1980) and are capable of mediating cytotoxic functions, including antibody-dependent cellular cytotoxicity (ADCC) (Poplack, et al, Blood 48, 809. 1976).

Assessment of the in vitro function of human monocytes has been hampered by a number of technical and theoretical problems. First, monocytes constitute a very low proportion of the cells in human peripheral blood (generally less than 5%); thus, obtaining large numbers of them has been quite difficult. In addition, very few techniques have emerged which allow for the large-scale isolation of purified populations of human monocytes by negative selection; instead, generally small numbers of rather impure monocytes are isolated on gradients such as Percoll (Hester, et al., 1981) or cells of higher purity are obtained by adhering them onto plastic or glass labware by positive selection (Werb. J. Exp. Med. 147, 1695. 1978).

When monocytes are obtained by positive selection, it may be difficult to remove them for further study; a variety of rather harsh measures are utilized to remove the adhered cells from the plastic or glass surface, ranging from the use of rubber policemen (Pennline, Manual of Macrophage Methodology, pp. 65-77. 1981), EDTA (Ackerman and Douglas, J. Immunol. 120, 1372. 1979), and lidocaine-containing solutions (Koski, et al, In Vitro Methods in Cell Mediated and Tumor Immunity, Academic Press, p. 359. 1976). The potential problems of adherence and positive selection are compounded when the cells are placed into culture since most researchers employ some sort of polystyrene labware to which the monocytes will readily adhere.

Adherent monocytes, of course, are in a different condition from their normal state of suspension in human peripheral blood. Therefore, the functions may also be different. Furthermore, when placed in medium conditions under which they are generally cultured, human monocytes demonstrate a number of theoretical and technical inadequacies. These problems mainly stem from the limited nutrients provided in most standard laboratory culture media for a cell as metabolically active as the human monocyte, and the additional potential artifacts created by culturing human monocytes with sera from different human individuals (AB serum) or from other species (such as fetal calf serum). Thus consistent and uniform conditions for culturing human monocytes cannot be assured from batch to batch.

In contrast, the present invention provides a precisely defined, serum-free medium and conditions for obtaining a large number, $10^8$ or more of highly enriched human monocytes from a single donor in long-term suspension culture. Such virtually unlimited supply of autologous human monocytes now makes it possible to use the monocytes for experimental, pharmaceutical and therapeutic purposes, particularly in immunotherapy.

Disclosure of the Invention

It is, therefore, an object of the present invention to provide isolated, substantially pure, human monocytes cultured in suspension in serum-free medium.

It is a further object of the present invention to provide a method for in vitro culturing and activating of purified human monocytes in suspension in a serum-free medium.

It is yet another object of the present invention to provide a pharmaceutical composition for immunotherapy in humans consisting essentially of isolated, purified human monocytes in suspension in a non-adherent container containing a suitable carrier, adjuvant and/or biological response modifier (BRM).

It is an additional object of the present invention to provide a method of treating cancer in mammals comprising administering to said mammal an immunotherapeutic amount of isolated, purified monocytes suspended in a serum-free medium with or without the presence of suitable adjuvants and/or biological response modifiers.

Other objects and advantages of the present invention would become apparent as the detailed description of the present invention proceeds.

Brief Description of the Drawings

These and other objects, features and many of the attendant advantages of the invention will be better understood upon a reading of the following detailed description when considered in connection with the accompanying drawings wherein:

Figure 1 shows a flow sheet of procedures involved in the monocyte-EVLA protocol for peritoneal colorectal carcinomatosis; and

Figure 2 shows distribution of $^{111}$indium-labeled activated monocytes in the peritoneum 4 hours after infusion into a peritoneal colorectal carcinomatosis patient.

Best Mode for Carrying Out the Invention

These and other objects and advantages of the present invention are achieved by providing isolated, substantially pure human monocytes cultured in suspension in a sterile, serum-free medium. The isolated, cultured human monocytes of the present invention may be fortified or activated with biological response modifiers and/or adjuvants.

An important aspect of the present invention is the use of such apparatus, containers, appliances, laboratory equipment and the like that are made of a material which is inert or non-toxic to the human monocytes and to which the human monocytes do not adhere or stick. The use of non-toxic, non-adherent wares throughout the manipulative steps, in any manner related to the handling of human monocytes, is a critical feature of the present invention. A preferred example of an inert, non-toxic, non-adherent and sterilizable material which can be suitably employed in accordance with the present invention is polytetrafluoroethylene (Teflon). Other similar or equivalent materials which would be apparent and suggested to one of ordinary skill in the art, can, of course, be also used so long as such material is non-toxic and non-adherent to the human monocytes. Teflon, however, is commercially and easily available (e.g. from DuPont Corporation, Wilmington, Delaware) and, therefore, is preferred.

The containers, in accordance with the present invention, are made out of solid Teflon sheets. Some examples of such containers or laboratory wares are flasks and micro titer plates and the like of various shapes and sizes, preferably ranging from 0.1 to 200 ml capacity. For convenience, of course, the shapes and sizes of solid Teflon containers are chosen to be similar to other laboratory wares routinely used for testing, culturing or other preparative work.

The term "substantially pure" or "substantially purified" as used herein means that the human monocytes are as pure as it is humanly possible to obtain by the techniques and methods commonly known to one of the ordinary skill in the art to which this invention pertains. However, a purity of 90 percent or greater is necessary for the monocytes to be substantially pure.

The term "activated" monocytes or "activation" of monocytes as used herein means that the isolated monocytes have been exposed to or treated with such agents or factors which would stimulate, modify or enhance the immunoregulatory, biological or physiochemical property or the native characteristics or functions of the monocytes. Such agents or factors include suitable antigens, adjuvants, biological response modifiers (BRMs) and the like.

The term "inert" container as used herein means that the material of which said container is made of has no deleterious or toxic effect on the natural or normal functions of the monocytes cultured in said container.

The term BRMs includes a diverse spectrum of compounds including natural cytokines such as interferons (IFN), lymphokines such as interleukin-1 (IL-1), certain synthetic chemicals with immunomodulatory properties such as polyriboinosinic acid, polyribocytidylic acid, poly L-lysine, carboxymethyl cellulose, (poly ICLC) and levamisole; immunomodulatory adjuvants such as bacille Calmette Guerin (BCG), Corynebacterium parvum, Staphylococcus protein A, monoclonal antibodies, tumor antigen preparations and the like. Colony stimulating factors (CSF) and prosta glandin E (PGE) are other examples of suitable BRMs.

Human monocytes can be isolated from the peripheral blood following routine techniques well known in the art. However, the preferred methods and materials employed for isolation and purification of the monocytes are described hereunder. Other preferred methods and materials utilized are also described. All publications cited hereunder are incorporated herein by reference.

## Isolation of Human Monocytes

Leukocytes were obtained by leukapheresis (Celltrifuge II leukapheresis apparatus. Travenol Laboratories, Deerfield, IL). Monocytes were then purified from the unfractionated mononuclear leukocyte preparation by counter-current centrifugation elutriation (CCE) (Stevenson and Fauci. Manual of Marcophage Methodology; Marcel Dekker, NY. pp. 75-80. 1981). The purity and viability of the monocyte preparations obtained were determined by nonspecific esterase staining, Wright's staining, and latex bead ingestion as described by Stevenson and Fauci, supra. The average yield of monocytes per normal donor was about 550 million cells (Stevens, et al. J. Immunol. Meth. 62, 353. 1983).

## Culture technique

New culture techniques were developed to allow for maintenance of the single-cell suspension state of purified human monocytes, utilizing specially developed culture plates made of Teflon (Corning Glass Works, Corning, NY). These culture plates match the exact dimensions of standard 24-well flat-bottomed polystyrene culture plates (Costar Plastics, Cambridge, MA). Human monocytes were counted with an automated cell counter (Electrozone/Celloscope, Particle Data, Elmhursts, IL) and were suspended in serum-free medium or (Roswell Park Memorial Institute, Buffalo, NY) RPMI 1640 + 10% human serum + L-glutamine at a concentration of $10^6$ monocytes/ml. For those studies in which human serum was employed, no improvement of BRM secretion was noted when concentrations in excess of 10% were utilized; therefore, 10% human AB in RPMI 1640 serum was used as the standard serum containing medium. No antibiotics were added to the cell culture suspension. The optimal concentrations of activators of CSF and PGE release were 100 $\mu$g/ml of muramyl dipeptide (MDP) (Calbiochem, La Jolla, CA), 5 $\mu$g/ml of lipopoly-saccharide (LPS) (Sigma, St. Louis, MO) and 50 $\mu$g/ml of polyriboinosinic acid polyribocytidylic acid (poly I:C) (Sigma). As an activator of interferon (IFN) secretion, an optimal final concentration of 200 $\mu$g/ml of poly I:C (Sigma) was added to the wells. Suspension monocyte cultures were placed at 37°C in a 5% $CO_2$ incubator. Maximal levels of the BRMs tested were found at the end of 48 h of culture, at which time the plates were spun once at 200 x g and the cell culture supernatant was harvested for subsequent determination of IFN, CSF or PGE activity. All cultures were performed in triplicate.

## Monocyte phagocytosis, retrieval and viability after culture

After 48 h of culture, various monocyte preparations were analyzed for viability by trypan blue dye exclusion, latex bead ingestion, and cell numbers remaining in culture. Latex beads followed by trypan blue dye were added to the cultures; aliquots of cells were aspirated from the Teflon suspension cultures after 30 min and assessed by phase microscopy for the percent viable and phagocytosing monocytes. For the adherent monocyte polystyrene culture plates, 100 cells were assessed in situ for phagocytosis and viability by inverted phase microscopy. Determination of the number of cells in suspension was performed by gently pipetting the culture well several times and counting the number of cells in the well aspirate. Determination of adhered cell numbers was made by vigorously scraping the aspirated well with a rubber policeman after the addition of 1 ml of RPMI 1640 and counting the cells dislodged.

## Serum-free medium

The cells were cultured in a serum-free medium as described by Brown, et al. (J. Cell. Physiol. 115, 191. 1983) consisting of Iscove's modified Dulbecco's medium (MDM) supplemented with human serum albumin (fatty acid free, 4 mg/ml), cholesterol (greater than 99% pure, 20 $\mu$g/ml), L-$\alpha$-phosphatidyl- choline (80 $\mu$g/ml), and human transferrin (98% pure; 1 $\mu$g/ml), all from Sigma Chemical Company; insulin (0.128U/ml, Eli Lilly and Co., Indianapolis, IN); ferrous chloride (7 x 10-11 M, Fisher Scientific Co., Fairlawn, NJ); and $\beta$-mercaptoethanol ($10^{-7}$ M, Eastman Kodak, Rochester, NY). The L-$\alpha$-phosphatidyl- choline and cholesterol were prepared together and sonicated with a Branson sonifier with a microtip at a setting of 6 for 60 min at 4°C. This preparation, like the others, was then filtered (0.45 um, Nalge Co. Rochester, NY) and stored at -20°C.

5

### Interferon assay

IFN activity of culture supernatants was determined (Biofluids, Rockville, MD) in microtiter plates by inhibition of the cytopathic effect of human foreskin cells infected with vesicular stomatitis virus (VSV) and is expressed in reference standard units. Reference hIFN-$\alpha$ was supplied by the National Institute of Allergy and Infectious Diseases.

### PGE radioimmunoassay

PGE was determined by radioimmunoassay (RIA) as described by Steel et al, (J. Allergy Clin. Immunol. 64, 287, 1979;) and Steel et al, (J. Biol. Chem. 256, 269. 1981). All of the results are expressed as pg PGE/ml culture supernatant.

### Colony stimulating factor (CSF) assay

CSF concentrations in stimulated monocyte supernatants were assayed as described by Ladisch et al, (Cancer Res. 39, 2544. 1979) and Schlick et al (Mechanisms of Immune Modulation, Marcel Dekker, NY, 1984). GM-CSF is expressed as U/ml, calculated from the number of myeloid colonies formed by $10^5$ normal bone marrow cells in the presence of 1.0 ml of the CSF source.

### Statistical methods

For each of the experiments, a linear statistical model which includes all of the main variables (plate type, medium type, activating agents and donors) along with main variable interactions was employed. Analysis of variance was performed on the raw CSF data, the natural (base e) logarithmic transformation of the IFN data and the stimulation indices (stimulated/control values) for the PGE data, to determine significance of main effects. For factors of more than 2 levels, Duncan's multiple range test was employed to determine significance of pairwise differences (Snedecor and Cochran, Statistical Methods, pp. 233-237. 1980).

### Analysis of culture system variables on human monocyte viability and numbers

Parallel comparative experiments were performed to analyze the function of human monocytes when cultured in Teflon plates versus polystyrene plates and in 10% AB serum versus serum-free medium. As shown in Table I, none of the culture conditions had any significant effect on the viability, phagocytosis, or overall cell retrieval of monocytes after 48 h of culture. It should be noted that while over 85% of monocytes cultured on polystyrene plates remained adhered to the bottom at 48 h, in the presence of 10% AB serum as well as in serum-free medium, in contrast over 90% of monocytes cultured in Teflon plates remained in suspension in both serum-containing and serum-free media after 48 h of culture.

### Effect of culture system variables on IFN release

When monocytes were exposed to an optimal concentration of poly I:C (200 $\mu$g/ml) (defined by dose-response curves) and the supernatants harvested at the optimal time point (48h), substantial differences were noted between paired samples cultured in AB serum versus serum-free conditions. In 5 separate experiments monocytes cultured in polystyrene plates demonstrated an increase in IFN release (P <0.01) under serum-free conditions (Table II), although individual-to-individual variation was noted in the levels of IFN released. A similar significantly enhanced IFN production in serum-free medium was noted when monocytes were cultured in Teflon plates. When parallel comparisons of IFN production by monocytes under the 2 culture plate conditions (Teflon versus polystyrene) were made (Table I), no difference in the effect of the culture vessel type on IFN production was found. These results clearly demonstrate that adherence was not required for monocyte IFN secretion.

### Effect of culture system variables on CSF release

The effects of serum-free versus AB serum and polystyrene versus Teflon culture plates are summarized in Table III. Serum-free medium was shown not to significantly affect the baseline level of CSF production in either the adherent or the suspension culture systems. In contrast, serum-free medium significantly enhanced CSF release (P <0.01) when optimal doses of LPS, MDP and poly I:C/LC were used (P <0.01).

TABLE I

Effect of Culture Variables on Viability,
Phagocytosis and Retrieval of Purified Human Monocytes

| | Freshly isolated | Polystyrene plates | | Teflon plates | |
|---|---|---|---|---|---|
| | | AB serum | Serum-free | AB serum | Serum-free |
| Wright's stain purity[a] | 91.4(1.5) | NT[c] | NT | NT | NT |
| Esterase stain positive[a] | 93.6(0.5) | NT | NT | NT | NT |
| Lates head ingestion[a] | 90.8(1.1) | 92.8(0.7) | 92.2(0.9) | 91.4(1.1) | 91.6(0.6) |
| Viability[a] | 99.6(0.2) | 97.6(1.0) | 99.0(0.3) | 98.0(0.3) | 97.6(0.4) |
| Number of cells in suspension[b] | 10.0 | 1.0(0.2) | 1.1(0.2) | 7.8(0.3) | 8.0(0.3) |
| Number of cells adhered[b] | 0 | 7.3(0.2) | 7.1(0.2) | 0.6(0.1) | 0.5(0.1) |
| Overall cell retrieval[a] | 100 | 8.2(3.6) | 80.8(3.7) | 85.4(3.1) | 85.0(2.5) |

a  Numbers denote mean percentage of 5 separate experiments, standard error is in parentheses.

b  Numbers denote mean (x $10^5$) of 5 separate experiments, standard error is in parentheses.

c  NT denotes not tested.

TABLE II

Poduction of IFN (U/ml. Expressed in
Natural Log [Base E] Values) by Cultured Monocytes

| Dose of stimulator | Experiment number | Polystyrene plates | | Teflon plates | |
|---|---|---|---|---|---|
| | | AB serum | Serum-free | AB serum | Serum-free |
| Control | 1-5 | ND[a] | ND | ND | ND |
| Poly 1:C (200 µg/ml) | 1 | 3.61+0.20[b] | 4.07+0.16 | 2.73+0.30 | 6.07+0.08 |
| | 2 | 5.22+0.20 | 6.14+0.31 | 7.52+0.53 | 7.45+0.31 |
| | 3 | 6.30+0.41 | 7.83+0.13 | 5.53+0.00 | 7.06+0.34 |
| | 4 | 6.14+0.15 | 6.45+0.00 | 5.68+0.16 | 6.22+0.40 |
| | 5 | 5.99+0.00 | 7.60+0.00 | 5.76+0.23 | 7.14+0.00 |

a  ND – note detectable.

b  Denotes mean ± standard error.

## TABLE III

### Secretion of CSF (u/m) by Cultured Monocytes

| Dose of stimulator | Experiment number | Polystyrene plates | | Teflon plates | |
|---|---|---|---|---|---|
| | | AB serum | Serum-free | AB serum | Serum-free |
| Control | 1 | 6.0± 4.0[a] | 25.0± 7.5 | 16.0± 4.5 | 23.0± 7.0 |
| | 2 | 8.0± 1.5 | 3.0± 0.5 | 6.0± 4.5 | 3.0± 0.5 |
| | 3 | 3.5± 1.5 | 5.0± 3.0 | 5.0± 2.0 | 3.0± 1.0 |
| LPS (5 ug/ml) | 1 | 134.0+18.5 | 227.0+19.0 | 142.0+12.0 | 282.0+18.5 |
| | 2 | 135.0+26.5 | 165.0+13.0 | 144.0+26.0 | 205.0+21.5 |
| | 3 | 130.0± 8.5 | 149.0± 1.5 | 130.0+10.5 | 152.0+14.5 |
| MDP (100 µg/ml) | 1 | 144.0+13.5 | 174.0+12.0 | 155.0+10.5 | 232.0+30.5 |
| | 2 | 114.0± 4.0 | 147.0+13.0 | 117.0+14.5 | 184.0+21.5 |
| | 3 | 102.0± 6.0 | 94.0+10.5 | 114.0± 9.0 | 120.0± 7.5 |
| Poly I C/LC (50 µg/ml) | 1 | 165.0+23.0 | 210.0+11.5 | 174.0+17.0 | 222.0+21.5 |
| | 2 | 162.0+16.0 | 160.0+10.5 | 162.0+16.0 | 200.0+16.0 |
| | 3 | 107.0± 4.0 | 112.0± 9.5 | 115.0± 7.5 | 109.0+7.0 |

[a] Mean ± standard error

It is to be noted that monocytes displayed a significant trend to more CSF release in Teflon culture plates (P <0.01) as compared to polystyrene plates under both AB and serum-free conditions.

Effect of culture system variables on PGE release

The most significant effect of serum-free medium on PGE release by human monocytes was a consistent lowering of the baseline rate of production (P <0.01). This lowered baseline allowed for a significantly higher stimulation index following poly I:C/LC, LPS and MDP stimulation (P <0.01) (Table IV). The stimulation index for PGE release was not significantly different when cultured in Teflon or polystyrene plates.

Ex Vivo Leukocyte Activation (EVLA) Therapy

The object of EVLA therapy is to remove substantial numbers of leukocytes from the peripheral blood or bone marrow of cancer or immune dysfunction patients, followed by the purification of certain cytotoxic leukocyte subsets. Such purified leukocytes are then expanded and/or activated to tumoricidal or immunotherapeutic activity in vitro followed by reinfusion or these cells into the sites of tumor burden or immune dysfunction in the patient.

The human blood monocyte and its differentiated tissue counterpart, the macrophage, are known to be capable of killing a wide range of tumor targets both

TABLE IV

Release of PGE (pg/ml) by Cultured Monocytes

| Dose of stimulator | Experiment number | Polystyrene plates | | Teflon plates | |
|---|---|---|---|---|---|
| | | AB serum | Serum-free | AB serum | Serum-free |
| Control | 1 | 6422.0+182.0[a] | 2207.0+ 70.0 | 8136.0+ 63.0 | 2313.0+345.0 |
| | 2 | 5773.0+ 67.0 | 1094.0+107.0 | 4361.0+111.0 | 871.0+26.0 |
| | 3 | 5382.0+ 81.0 | 453.0+104.0 | 7264.0+270.0 | 532.0+34.0 |
| LPS (5 µg/ml) | 1 | 17127.0+ 29.0(2.66)[b] | 2728.0+124.0(1.23) | 14721.0+782.5(1.81) | 1915.0+182.0(0.84) |
| | 2 | 7305.0+ 60.0(1.27) | 2114.0+ 59.0(1.96) | 6960.0+ 56.0(1.60) | 1487.0+ 55.0(1.7) |
| | 3 | 9505.0+183.0(1.76) | 2211.0+152.0(5.11) | 8367.0+113.0(1.15) | 2242.0+ 67.0(4.24) |
| MDP (100 µg/ml) | 1 | 10170.0+ 44.0(1.58) | 3976.0+ 44.0(1.81) | 11806.0+617.5(1.45) | 3144.0+277.5(1.37) |
| | 2 | 6641.0+ 40.0(1.15) | 2064.0+ 79.0(1.91) | 8367.0+121.0(1.92) | 3498.0+ 60.0(4.02) |
| | 3 | 6394.0+365.0(1.18) | 2022.0+ 31.0(4.74) | 4887.0+ 44.0(0.67) | 2329.0+ 73.0(4.40) |
| Poly I:C/LC (50 µg/ml) | 1 | 11054.0+ 95.0(1.72) | 3027.0+406.0(1.37) | 9718.0+ 47.0(1.19) | 2842.0+160.5(1.25) |
| | 2 | NA[c] | NA | NA | NA |
| | 3 | 9278.0+247.0(1.72) | 3005.0+ 48.0(7.04) | 5474.0+ 52.0(0.75) | 4705.0+ 82.0(8.91) |

a Denotes mean + standard error.

b Numbers in parentheses indicate stimulation index

c NA denotes PGE release in response to poly I:C/LC not assayed.

directly and by antibody-dependent cellular cytotoxicity (ADCC) mechanism. (Keller, Immunobiology of the Macrophage. NY:Academic Press, 487-508. 1976; Hibbs, The Macrophage in Neoplasia. NY:Academic Press, 83-98. 1976; Evans, Immunobiology of the Macrophages. NY:Academic Press, 535-576. 1976; Haskill, Int. J. Cancer. 20:432-440. 1977). However, heretofore no EVLA trials using monocytes have been performed.

Several clinically relevant criteria must be considered in the design of any EVLA protocol. As shown in Table V, it is important to employ a single, purified, sterile, toxic-free, cytotoxic leukocyte subset that is capable of being used in a clinical setting (this included being not only sterile but devoid of any toxins). The use of purified leukocyte subsets allows for precise toxicity and physiologic determinations for each cell type. In the event that the administration of a single cell type is not ameliorative or curative of the immune dysfunction then one can build upon the single cell type baseline clinical data to design rational "combination EVLA" protocols.

It is important that a sufficient number of cells be obtained to produce a clinical effect when infused into the patients (at least 500 million). It is also essential that these cells be maintained in a state of suspension to avoid the clinical problems encountered when trying to infuse clumps of cells. Leukocyte activating substances must be of clinical grade. Until graft-versus-host disease problems have been clinically minimized, it is preferred that EVLA protocols are

Table V. Desirable Attributes of an EVLA Therapy Protocol.

A. A single purified cytotoxic effector cell should be employed.
B. Should be able to purify effector cells in a manner that allows cells to be used clinically (i.e., pyrogen, pathogen and toxin-free).
C. If amplifying and/or activating substances are employed, they should be purified and of clinical grade (IND # required).
D. FDA will require a separate approval for the "activated effector cell" prior to patient use.
E. Cells should be capable of being cultured in suspension--in absence of antibiotics, animal sera or any other sensitizing agents.
F. Mechanism for obtaining enough effector cells to elicit clinical response and/or toxicity should be identified.
G. Autologous cells should be employed until graft-vs-host disease problem is solved.
H. Mechanism for homing effector cells to tumor site must be identified.
I. In vitro testing should demonstrate activity of purified effector cells against patient's tumor cell type.

restricted to the use of autologous leukocytes. A further consideration is that if in vitro testing demonstrates that the patient's leukocytes have activity against his or her tumor cells, mechanisms must be devised to allow for "homing" of the cytotoxic leukocytes to the sites of tumor burden in the patient.

The Monocyte-EVLA Protocol for Peritoneal Colorectal Carcinomatosis:

As mentioned herein supra, a great number of the immunologic effector cell functions of monocyte/macrophage cell types have been characterized including antigen presentation, the production of certain immunoregulatory biological response modifiers such as alpha interferon, interleukin 1, colony stimulating factor, and certain critical components of the complement system. Monocytes and macrophages are also believed to be the predominant cellular component in a number of cell-mediated immune responses including granuloma formation. The monocytes and macrophages are also known to be phagocytic and because they express Fc receptors for immunoglobulin G, they are major participants in antibody-dependent cellular cytotoxicity reactions (ADCC).

It is only recently, that monocytes have been characterized as potent tumoricidal effector cells. Human monocytes have an ability to recognize and kill tumor targets in vitro that is independent of antibody and may be augmented by such agents as interferon and muramyl dipeptide. Monocytes and macrophages are major components of the cellular infiltrate of both rodent and human tumors; in vitro studies using tumor-associated macrophages from both human and rodent tumors indicate that these cells can be activated to tumoricidal activity with various biological response modifiers. The reproducible in vitro activity of human blood monocytes, in the suspension culture system as described herein, is of course, indicative of clinical utility.

The present study is the first to demonstrate clinical feasibility of EVLA therapy.

As shown in Table VI a number of technical and logistic difficulties relevant to the handling of human blood monocytes in the EVLA protocol setting have now been solved. A new technique as described herein for isolating highly purified blood monocytes in large numbers by a combination of two blood component separation techniques: cytapheresis (Stevenson et al, Plasma Ther Transfus. Technol. 4:57-63. 1983, Fenwal Laboratories, Deerfield, IL), and counter-current centrifugal elutriation (Stevenson, Methods in Enzymology:Immunochemical Techniques, Part G. NY:Academic Press. Beckman Laboratories, Palo Alto, CA) has been developed. Using a combination of these techniques, in accordance with the present invention, it has now been possible, to obtain greater than $10^9$ human monocytes with a purity of 90 percent or more by a negative selection process that allows the cells to remain in suspension. The cells thus obtained are sterile and devoid of antibiotics or any toxins. Utilizing the methodology described herein supra,

Table VI EVLA Therapy with Monocytes

A. Techniques for obtaining purified effector monocytes identified; counter-current centrifugal elutriation.
B. Monocyte purification and activation techniques leave cells pyrogen, pathogen and toxin free.
C. Purified, clinical-grade monocyte activating substances (gamma IFN) available.
D. FDA approval for purifed gamma interferon activated monocytes-granted.
E. Capable of culturing monocytes in suspension without antibiotics, animal sera or other sensitizing agents.
F. Mechanism for obtaining enough monocytes for clinical effect identified; counter-current centrifugal elutriation.
G. Autologous monocytes can be used in an EVLA setting.
H. Mechanism for "homing" activated monocytes to sites of patient tumor burden identified: the peritoneal colorectal carcinomatosis.
I. In vitro testing of monocyte cytotoxicity against various human tumor cell types--ongoing.

sufficient autologous monocytes from a single patient are obtained to demonstrate a significant clinical effect. Moreover, the ability to "home" these tumoricidal cells to the site of tumor burden has also been demonstrated in patients with peritoneal colorectal carcinomatosis (Table VII).

Peritoneal-colorectal carcinomatosis disease represents a metastatic form of colon cancer and is universally fatal. No effective therapy presently exists. It is believed that the disease tends to metastasize to distant organs (such as lung and bone) in the late stage and frequently kills the patient by direct local extension into the viscera. This tendency to remain localized suggests the possibility that local elimination of this metastatic disease may greatly improve the length and quality of patient life. Previous studies with these patients in which Tenckhoff catheters have been inserted into the peritoneal space for the instillation of 5 fluorouracil ("5-FU bellywash" protocol), (Sugarbaker, Principles and Practice of Oncology. Philadelphia:Lippinicott Co., 1982 have provided an excellent clinical background for the direct deposition of tumoricidal leukocytes into the site of tumor burden in these patients.

## TABLE VII

### Monocyte-EVLA Protocol for Peritoneal Colorectal Carcinomatosis

| | |
|---|---|
| Week 0 | Debulking surgery/insertion of Tenckhoff catheter |
| | ↓ |
| | Patient cytapheresis/elutriation of monocytes |
| | ↓ |
| Weekly x 16 weeks | Overnight activation of monocytes in gamma interferon |
| | ↓ |
| | Infusion of activated monocytes intraperitoneally |
| | ↓ |
| Week 16 | "Second-look" laparotomy |
| | ↓ |
| Weeks 17-41 | Maintenance EVLA therapy (if indicated) |

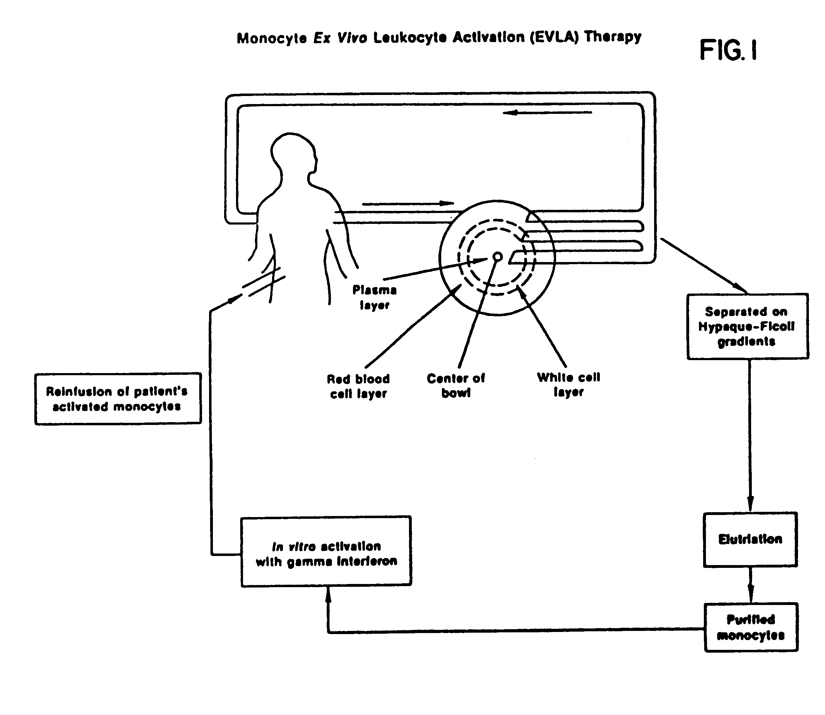

The monocyte EVLA protocol for peritoneal colorectal carcinomatosis, in accordance with the present invention, is conducted at the National Cancer Institute, Bethesda, MD. As shown in Figure 1, patients with a diagnosis of peritoneal colorectal carcinomatosis are referred to the National Cancer Institute for debulking surgery to render the patients as disease-free as is surgically possible, coupled with the insertion of a Tenckhoff catheter which communicates with the peritoneal space. Immediately following surgery, the patient has the intraperitoneal infusion of approximately 500 to 900 million gamma interferon activated purified blood monocytes. These cells are obtained by a 2-hour cytapheresis procedure followed by purification of the monocytes by counter-current centrifugal elutriation. Following this, the purified monocytes are cultured in suspension overnight in gamma interferon (Immunomodulator Laboratories, Stafford, TX) at a concentration of 1000 U/ml. After overnight activation in gamma interferon, the monocytes are infused into the peritoneal space via the Tenckhoff catheter. In addition, the patient receives a 2-liter infusion of peritoneal lavage fluid such as Impersol, (Travenol Laboratories, Deerfield, IL) to allow for the maximal distribution of the patient's activated monocytes throughout the peritoneal space. In order to determine with certainty the patient's response to this form of therapy, a "second-look" laparotomy at the conclusion of 16 weeks of monocyte EVLA is conducted. Patients who are found to have a complete or partial response to monocyte EVLA therapy are then offered a 6-month maintenance monocyte EVLA therapy regimen.

Clinical Observations Regarding the Monocyte-EVLA Protocol:

Initially, two patients were treated in the monocyte EVLA protocol at the National Cancer Institute at the Biological Therapeutics and Surgery Branches. Both patients were remarkably similar in the nature of their disease and their response to monocyte EVLA therapy. Both were white females, 38 and 41 years of age; one had a diagnosis of peritoneal colorectal carcinomatosis for 12 months, the other for 6 months. Both patients were without evidence of distant metastatic disease and neither patient had any other serious illnesses. Their functional status was excellent. Both patients had severe involvement of the peritoneal surfaces with cancer; virtually no aspect of the parietal peritoneum was spared. However, both patients had visibly less metastatic disease on the small intestine than elsewhere. Attempts to remove as much grossly visible disease as possible were successful. One patient required removal of a large segment of the colon in order to dissect away the malignancy. The other patient required removal of the spleen for the same purpose.

Both patients tolerated the monocyte EVLA therapy remarkably well. As has been learned from the second patient, this therapy is safely performed in the outpatient department following the patient's recovery from the initial debulking surgery. Typically, the patients arrive one morning in the cytapheresis unit outpatient department and undergo a 2-hour cytapheresis procedure to remove between 5 and $9 \times 10^9$ leukocytes. Following this procedure they are sent home, and that afternoon and evening the patients' monocytes are purified by elutriation and placed in suspension culture with gamma interferon (1000 U/ml). The next morning, the patients return to the outpatient department to receive the infusion of their activated blood monocytes along with 2 litres of additional intraperitoneral Impersol; this infusion generally takes approximately 30 minutes. The patients then return home with oral analgesics and Tylenol. Generally, within 4 to 6 hours of the infusion of the monocytes, the patients note the onset of a low-grade fever (consistently less than 101° F) and a low-grade abdominal pain. The fever is manageable with Tylenol, and the pain is usually manageable with oral analgesics (occasionally patients have received parenteral narcotics in the outpatient department if the pain was too severe). Within 12 hours, both the low-grade fever and abdominal pain had generally subsided, and both patients had spent the rest of the week performing their normal daily activities. In both patients, a low-grade granulocytopenia after the first 4 to 7 cytapheresis procedures (total leukocyte count approximately 3,500) is noted; at this juncture, the frequency of the EVLA treatments is adjusted to once every other week with normalization of the peripheral leukocyte count.

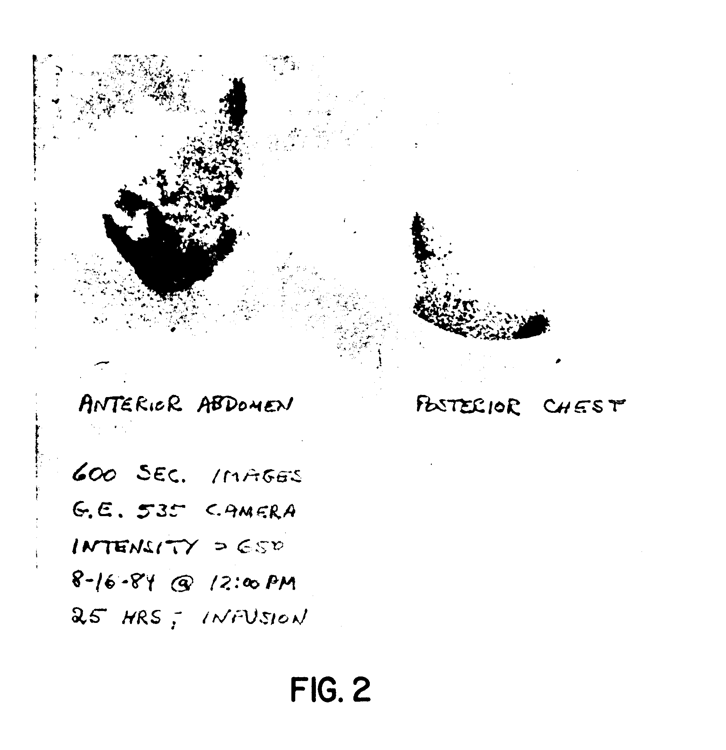

Midway through the protocol, the trafficking pattern of the intraperitoneally infused monocytes by prelabeling them with [111]indium is analyzed. Figure 2 shows the typical distribution of the [111]indium-labeled monocytes throughout the peritoneal space. The distribution is homogeneous with the few patchy areas of decreased uptake shown at second-look surgery to be due to postoperative adhesions. When interpretable images from these patients up to 5 days after intraperitoneal infusion of $^{111}$In-labeled monocytes; is obtained, the evidence indicates that these cells do not traffic outside of the peritoneal space. Instead, they appear to become incorporated in the cellular matrix of the peritoneum, most likely transforming into tissue macrophages.

Both of the above-cited patients have undergone the "second-look" laparotomy staging procedure. Both were found to have normalization of the majority of the surface of their peritoneum including the sites most heavily infiltrated with tens of thousands of metastatic lesions at the first surgery. Neither of the patients were found to have any bulky lesions of the colon or the viscera, nor were they found to have distant metastases. However, they both had very small amounts of residual disease in places which were restricted (predominantly by peritoneal adhesions) from access to the monocytes. They were rendered disease-free by removing the adhesions, exposing the area, and surgically excising the lesions (all < 1 cm). Both patients then went on to receive maintenance monocyte EVLA therapy following recuperation from the "second-look" laparotomy procedure.

The results noted above clearly indicate that immunotherapy of peritoneal colorectal carcinomatosis with activated blood monocytes is a feasible procedure. In order to obtain maximal antitumor effect the activated monocytes can be combined with other factors such as natural killer lymphocytes, antigen-specific killer T lymphocytes, B-cells, and the like. Such "combination EVLA" therapy can replace, supplement, mimic or reconstruct the natural immunologic system.

Of course, the availability of in vitro cultured, autologous, monocytes in suspension opens a new vista for immunotherapy and/or fortification and supplementation of immune regulatory system in the mammals where such system has been adversely effected due to immune dysfunction.

It is understood that the examples and embodiments described herein are for illustrative purposes only and that various modifications or changes in light thereof will be suggested to persons skilled in the art and are to be included within the spirit and purview of this application and the scope of the appended claims.

## Claims

**Claims for the following Contracting States: BE, FR, IT, GB, DE, CH, LI, SE, NL, LU**

1. A pharmaceutical composition for immunotherapy in humans consisting essentially of an immunotherapeutic amount of isolated, at least 90% pure, functional human monocytes cultured in suspension and a sterile pharmaceutically acceptable carrier.

2. The composition of claim 1, wherein said monocytes are activated monocytes.

3. The composition of claim 2 further comprising an activating amount of a biological response modifier.

**Claims for the following Contracting State: AT**

1. A method of preparing a pharmaceutical composition for immunotherapy in humans which comprises suspending an immunotherapeutic amount of isolated, at least 90% pure, functional human monocytes cultured in suspension in a sterile pharmaceutically acceptable carrier.

2. The method of claim 1 wherein said monocytes are activated monocytes.

3. The method of claim 2 which comprises adding to the suspension an activating amount of a biological response modifier.

## Revendications

**Revendications pour les Etats contractants suivants: BE, FR, IT, GB, DE, CH, LI, SE, NL, LU**

1. Composition pharmaceutique pour effectuer une immunothérapie chez l'homme, comprenant principalement une quantité immunothérapeutique de monocytes humains fonctionnels, isolés et purs à au moins 90 %, cultivés en suspension, et un véhicule stérile pharmaceutiquement acceptable.

2. Composition selon la revendication 1, dans laquelle les monocytes sont des monocytes activés.

3. Composition selon la revendication 2, comprenant en outre, une quantité activatrice d'un modificateur de réponse biologique.

**Revendications pour l'Etat contractant suivant: AT**

1. Procédé de préparation d'une composition pharmaceutique pour effectuer une immunothérapie chez l'homme, qui comprend la mise en suspension d'une quantité immunothérapeutique de monocytes humains fonctionnels, isolés et purs à au moins 90 %, cultivés en suspension, dans un véhicule stérile pharmaceutiquement acceptable.

**EP 0 205 387 B1**

**2.** Procédé selon la revendication 1, dans lequel les monocytes sont des monocytes activés.

**3.** Procédé selon la revendication 2, qui comprend l'addition à la suspension d'une quantité activatrice d'un modificateur de réponse biologique.

**Patentansprüche**
**Patentansprüche für folgende Vertragsstaaten: BE, FR, IT, GB, DE, CH, LI, SE, NL, LU**

**1.** Pharmazeutische Zusammensetzung für die Immuntherapie von Menschen, bestehend im wesentlichen aus einer immuntherapeutischen Menge isolierter, mindestens 90 % reiner, funktioneller menschlicher Monozyten, kultiviert in Suspension, und einem sterilen, pharmazeutisch verträglichen Träger.

**2.** Zusammensetzung nach Anspruch 1, wobei die Monozyten aktivierte Monozyten sind.

**3.** Zusammensetzung nach Anspruch 2, weiterhin umfassend eine aktivierende Menge eines eine biologische Antwort modifizierenden Mittels.

**Patentansprüche für folgenden Vertragsstaat: AT**

**1.** Verfahren zum Herstellen einer pharmazeutischen Zusammensetzung für die Immuntherapie von Menschen, umfassend das Suspendieren einer immuntherapeutischen Menge isolierter, mindestens 90 % reiner, funktioneller menschlicher Monozyten, kultiviert in Suspension, in einem sterilen, pharmazeutisch verträglichen Träger.

**2.** Verfahren nach Anspruch 1, wobei die Monozyten aktivierte Monozyten sind.

**3.** Verfahren nach Anspruch 2, umfassend das Zusetzen einer aktivierenden Menge eines eine biologische Antwort modifizierenden Mittels zu der Suspension.

Monocyte *Ex Vivo* Leukocyte Activation (EVLA) Therapy  FIG. I

Plasma layer

Red blood cell layer

Center of bowl

White cell layer

Separated on Hypaque-Ficoll gradients

Reinfusion of patient's activated monocytes

*In vitro* activation with gamma interferon

Elutriation

Purified monocytes

EP 0 205 387 B1

ANTERIOR ABDOMEN          POSTERIOR CHEST

600 SEC. IMAGES
G.E. 535 CAMERA
INTENSITY = 650
8-16-84 @ 12:00 PM
25 HRS ; INFUSION

FIG. 2